# EUROPEAN PATENT APPLICATION

(11) **EP 2 827 138 A1**
(43) Date of publication of application: **21.01.2015**
(21) Application number: 12871407.8
(22) Date of filing: 15.06.2012
(51) Int. Cl.: G01N 27/26, C12Q 1/68, G01N 33/487

(54) **METHOD FOR CARRYING OUT SPEED REDUCING AND MONOMER CATCHING ON NUCLEIC ACID MOLECULE BASED ON SOLID STATE NANO-PORE**

(30) Priority: 13.03.2012 CN 201210065833
(71) Applicant: President and Fellows of Harvard College, Cambridge, MA 02138 (US); Peking University, Beijing 100871 (CN)
(72) Inventor: LU, Bo, Beijing 100871 (CN); ZHAO, Qing, Beijing 100871 (CN); YU, Dapeng, Beijing 100871 (CN); HOOGERHEIDE, David, P., Cambridge, MA 02138 (US); GOLOVCHENKOC, Jene, A., Cambridge, MA 02138 (US)
(74) Representative: Lahrtz, Fritz
(86) International application number: PCT/CN2012/000840
(87) International publication number: WO 2013/134902

(57) **Abstract**

Disclosed is a method for carrying out speed reducing and monomer catching on a nucleic acid molecule based on a solid state nano-pore. The method comprises the following steps: adding nucleic acid molecules to be determined into a nano-pore sequencing device filled with electrolyte, the nano-pore sequencing device comprising an electrolytic tank having a positive pole and negative pole, and a solid state nano-pore film separating the positive pole and negative pole of the electrolytic tank; placing the nucleic acid molecules into a positive pole chamber of the electrolytic tank, and introducing a pressure external field into the positive pole chamber to serve as a driving external field for the punching of the nucleic acid molecules while determining; applying a voltage between the positive pole and negative pole to serve as an electric field external field opposite to the pressure external field; according to the purposes of speed-reducing or catching during determining, making the acting force of the pressure external field on the nucleic acid molecule in the nano-pore larger than or approximately equal to the electric field force. On the premise of not reducing the signal to noise ratio of a measuring signal, the method can effectively reduce the speed at which the DNA molecules pass through a nano-pore component, thereby slowing down the punching rate by more than one order of magnitude.

## Description

### FIELD

This invention relates to a method to slow down nucleic acid molecules and realize single-molecule capture based on solid-state nanopores.

### BACKGROUND

DNA single-molecule detection based on solid-state nanopore devices has become one of the most promising candidates in future third generation fast and cost-effective human gene sequencing, which aims to fulfill one person genome sequencing in 24 hours by less than 1000 US dollars. It has been a very hot topic in research and application explorations. Besides Harvard University, Boston University, Brown University, California Institute of Technology, and Delft University of Technology, IBM claims to join the next generation genome sequencing (1000$) plan in October 2009,indicating that the technique to realize sequencing based on nanopore devices has turned from fundamental research into application research. (D. Branton, et al. Nature Biotechnology 26 (10), 1146 (2008); M. Zwolak and M. Di Ventra, Reviews of Modern Physics 80 (1), 141 (2008).) Electrophoresis-driving of molecules to translocate through a nanoscale pore in ionic solution can realize single molecule detection and analysis. In this tiny space of nanopores, one can use various methods to analyze large quantity of molecules in a short time. When the polymer molecules translocate through nanopores, the detected signal characteristic corresponds to their structure information, which can directly characterize thousands base pair single strand DNA molecule. This can avoid using sample amplification or labeling, making fast and low cost DNA sequencing possible. Right now the biggest remaining challenge is that the DNA translocation speed through nanopores is too fast to reach the device detection limit (low time resolution), which can not realize base pair distinction. How to use solid-state nanopores to realize DNA molecule trap, is very important to study single-molecule capture, manipulation, control in solution. Besides, it is significant to investigate some biological processes such as, the chemical transformation, molecule internal dynamic changes.

In a typical setup, an external electric field drives a DNA molecule through a nanometer-size pore in a thin synthetic membrane, producing a characteristic temporary drop in the trans-pore ionic current due to ion flux blockage when DNA is occupied in the nanopore. This ionic current blockage and time duration of such current drop corresponds to the biological information of the translocated DNA molecule. The DNA translocation speed (1bp/µs) is too fast to meet the bandwidth limits (4µs) to distinguish base pair differences using conventional voltage driving techniques. In order to improve the temporal resolution, we need to increase the DNA translocation time, and effectively slow down the DNA translocation speed inside nanopores. Amit Meller et al. from Boston University used different salt gradients across nanopores in the cis-chamber and trans-chamber of a tiny nanopore (∼5nm) to slow down DNA translocation. (M. Wanunu, W. Morrison, Y. Rabin, A. Y. Grosberg and A. Meller, Nature Nanotechnology, 5, 160, (2010)) Nevertheless, they did not give a clear explanation, plus because the nanopore they used is very small, the much slower translocation time may result from the DNA-nanopore interaction, which is difficult to be controlled in experiments. Very small nanopores are rather difficult to fabricate with very low success rate.

Jiali Li et al. from University of Arkansas introduced glycerol into solution to increase the event duration time. (D. Fologea, J. Uplinger, B. Thomas, D. S. McNabb, and J. L. Li, Nano Letters 5, 1734 (2005)) Adding glycerol causes the increase of solution viscosity, which decreases the current blockage magnitude, resulting in a lower signal-to-noise ratio. In addition, decreasing voltage can cause slower translocation, but decrease the current signal as well, to worsen the signal-to-noise ratio. Many methods to slow down DNA translocation are realized on the basis of sacrificing the signal-to-noise ratio, which make it difficult to precisely measure signal corresponding to related biological information.

In previous work with solid-state nanopores, the DNA single-molecule capture dynamics in solution could not be studied directly because the location of the molecule was unknown and difficult to detect until it entered the nanopore. DNA has a very fast translocation speed under electric field in solution, and its move trajectory after it translocates through nanopores is unknown. Therefore, it is very difficult to realize DNA molecule re-capture. Prof. Jene Golovchenko's group from Harvard University developed an experimental system similar as a "molecule Ping Pang" to recapture and trap single molecules. (M. Gershow, J. A. Golovchenko, Nature Nanotechnology, 2, 775 (2007)) They make DNA translocate through nanopore first by electric field, then reverse the electric field direction very fast within (2-32 ms) to recapture the DNA molecule for a reversal translocation. Repeated reversal electric field direction can realize the same molecule capture many times in a certain period of time. The method needs very high experimental requirements, such as circuits design, programming, and the success rate is not very high. The event duration time is very short (<ms) when DNA is recaptured, making it difficult to achieve single-molecule manipulation, detection and analysis inside nanopores.

Prof. Cees Dekker's group from Delft University of Technology used optical tweezers to trap DNA. (Keyser, U. F., B. N. Koeleman, S. V. Dorp, D. Krapf, R. M. M. Smeets, S. G. Lemay, N. H. Dekker, and C. Dekker, 2006, Nat. Phys. 2, 473) They used infrared laser as optical trap to manipulate DNA-coated polymer bead. Optical tweezers was used to control the motion of the bead, to trap DNA into nanopores. Signal obtained via the technique has big noise with rather complicated experimental setup.

### PURPOSES OF THE INVENTION

One purpose of this invention is to provide a method for slowing down the nucleic acid molecule translocation in nanopore sequencing.

This method, which helps slowing down the nucleic acid molecule translocation, is based on the solid-state nanopore sequencing technique with the following procedures: inject the nucleic acid molecule sample into the electrolyte-filled nanopore sequencing device. And the nanopore sequencing device mentioned above is made up of two electrolytic cells, each with a positive and negative electrode, and one piece of solid-state nanopore membrane in between to separate those cells apart. The above device is improved as follows: we inject the nucleic acid molecule sample into the cis side of electrolytic cells, introduce external pressure as the force field to drive the nucleic acid molecule pass through the solid-state nanopore. Meanwhile, voltage bias is applied across the nanopore as balance force field against the external pressure, and the force acted on nucleic acid molecule in solid-state nanopore by external pressure is larger than by electric field.

Nucleic acid molecule mentioned above includes double-strand DNA, single-strand DNA, RNA and PNA.

The external pressure can be adjusted in the range from 1.6 to 2.6 standard atmospheric pressure, and the voltage bias can be changed from 40 to 160 mV.

According to the translocation time we need, external pressure and voltage bias can be adjusted among those above ranges, leading to the perfect condition of pressure and bias for slowing down the translocation speed.

During detection, considering different electrolyte and nanopore state, concentration of the electrolyte varies from 0.1 to 3.2 mol/L, while the pH from 8 to 10. KCl solution is usually used as the electrolyte, other solutions like NaCl and LiCl can be selected as well.

The solid-state nanopore fabricated in the membrane is from 10 to 20 nm in diameter and 20 to 100 nm in depth.

The other purpose of this invention is to provide a method for single molecule trap of the nucleic acid molecules, also based on nanopore sequencing technique. The trap behavior happened on molecule partly tethering on membrane surface and partly driving by the pressure-derived force and voltage-derived force. The state of such tethering molecule whether is captured inside the nanopore or repulsed out from nanopore depends on the resultant force direction.

The procedures are as follows: inject the nucleic acid molecule sample into the electrolyte-filled nanopore sequencing device. And the nanopore sequencing device mentioned above is made up of two electrolytic cells, each with a positive and negative electrode, and one piece of solid-state nanopore membrane in between to separate those cells apart. The above device is improved as follows: we inject the nucleic acid molecule sample into the cis side of electrolytic cells, introduce external pressure as the force field to drive the nucleic acid molecule pass through the solid-state nanopore. Meanwhile, voltage bias is applied across the nanopore as balance force field against the external pressure, and the resultant force acted on nucleic acid molecule in solid-state nanopore by the balance of external pressure and electric field is nearly 0.

Nucleic acid molecule mentioned above includes double-strand DNA, single-strand DNA, RNA and PNA.

The external pressure introduced is adjustable from 1.6 to 2.6 standard atmospheric pressure, and the voltage bias can be changed from 40 to 160 mV.

According to the translocation time we need, external pressure and voltage bias can be adjusted among those above ranges, leading to the perfect condition of pressure and bias for slowing down the translocation speed.

During detection, considering different electrolyte and nanopore state, concentration of the electrolyte varies from 0.1 to 3.2 mol/L, while the pH from 8 to 10. KCl solution is usually used as the electrolyte, other solutions like NaCl and LiCl can be selected as well.

The solid-state nanopore fabricated in the membrane is from 10 to 20 nm in diameter and 20 to 100 nm in depth.

The last purpose of this invention is to provide a method for detecting and resolving shorter molecules by reducing translocation speed, also based on nanopore sequencing technique.

The procedures are as follows: inject the nucleic acid molecule sample into the electrolyte-filled nanopore sequencing device. And the nanopore sequencing device mentioned above is made up of two electrolytic cells, each with a positive and negative electrode, and one piece of solid-state nanopore membrane in between to separate those cells apart. The above device is improved as follows: we inject the nucleic acid molecule sample into the cis side of electrolytic cells, introduce external pressure as the force field to drive the nucleic acid molecule pass through the solid-state nanopore. Meanwhile, voltage bias is applied across the nanopore as balance force field against the external pressure, and we successfully achieve translocation, capture, release and reverse translocation of the nucleic acid molecule by adjusting the direction of resultant force acted on the molecule in solid-state nanopore of external pressure and electric field.

This invention, which is based on nanopore sequencing technique, introduces external pressure as the force field to drive DNA molecule pass through the solid-state nanopore while the voltage bias is applied as the balance force. As a result, the translocation speed of DNA molecule through a 10 nm diameter solid-state nanopore in the SiN membrane is obviously slowed down to an order of magnitude or more, improving the time resolution of DNA sequencing in a large scale, while the SNR (signal to noise ratio) is maintained as before. Besides, the fact that this invention gets rid of the uncontrollable interaction problem without using extremely small nanopore (less than 5 nm in diameter), makes it easier to run the DNA sequencing experiment and improves the repeatability and controllability compared with current technique. Furthermore, by adjusting suitable solution concentration, voltage bias and external pressure, short strands of DNA molecule that are less than 1 kb (kilo-base pair) which never reported before by current nanopore sequencing technique with the same diameter, can be now easily detected in this invention.

By means of adjusting the intensity of external pressure, the duration time of captured DNA molecule in solid-state nanopore is significantly extended by 4 to 5 orders of magnitude, to as large as dozens of seconds, which means an exactly single molecule trap. This result plays an important role in single molecule study like capture, detection and analysis. Meanwhile, the experiment device is easily assembled and has advantages of high controllability, repeatability and signal to noise ratio.

Compared to current DNA molecule detecting technique according to reports in literatures, the advantages of this invention can be described as follows:
1. DNA molecule translocation speed through nanopore is significantly slowed down while the time resolution is improved as well by means of introducing external pressure as driving force and voltage bias as slowing down force. So far, this high time resolution cannot be reached by other methods under the premise of staying with high SNR.
2. As the result of introduced external pressure, there is no need for this invention to turn down the voltage bias to slow down a DNA molecule, which helps stay with high SNR.
3. Compared with other slowing down methods, this invention only uses an extra pressure meter and a Nitrogen tank that are connected to a standard nanopore DNA sequencing device to introduce external pressure. This method has nice repeatability and doesn't acquire complex process or a master hand, which is good for improving the success rate and efficiency of experiment.
4. Short strands of DNA molecule like 3 kb, 1 kb, and even 600 bp can be successfully detected while DNA of 3 kb length or less is hardly detected by normal techniques in the absence of external pressure. The fact above enlarges detection range of DNA by solid-state nanopore in a large scale, which lays a solid foundation for achieving exactly DNA sequencing in the future.
5. A DNA molecule can be trapped and its motion direction also can be controlled by adjusting the balance of pressure and electric force. The ultralong capture time, which extends current technique by 4 to 5 orders of magnitude, plays an important role in the study of DNA molecule dynamics. For example, molecule structure, chemical reactive state and other bio-related information can be detected and analyzed.

### FIGURES

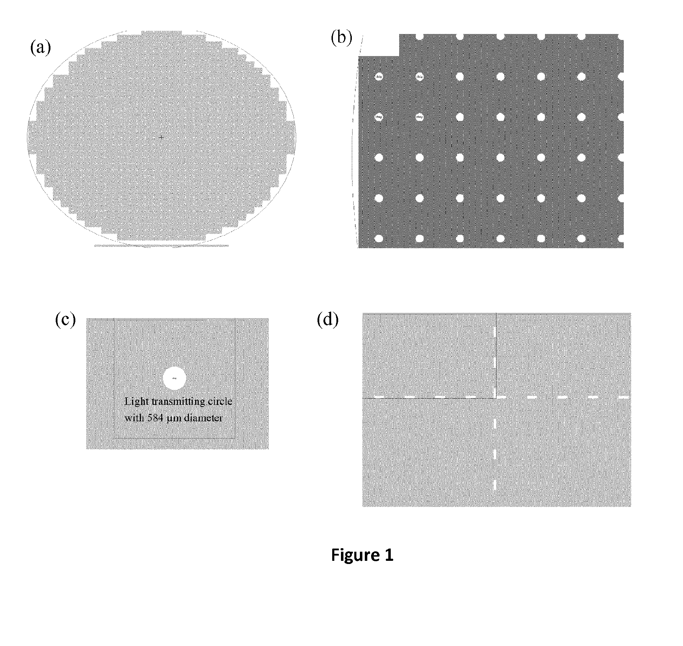

Figure 1 Schematic figure of the lithography mask for the solid-state nanopore of this invention. (a) The lithography mask edition graphics for 4 inch silicon wafer. (b) Partial enlarged detail of Figure.1a, white circles represent the circular light transmission area of each 3 mm*3 mm chip. (c) Further enlarged view of Figure.1b, the circular transmission area of each 3 mm*3 mm chip can by clearly observed. (d) White dotted lines represent saw lane, which helps small chips to be cleaved from the 4 inch silicon wafer.

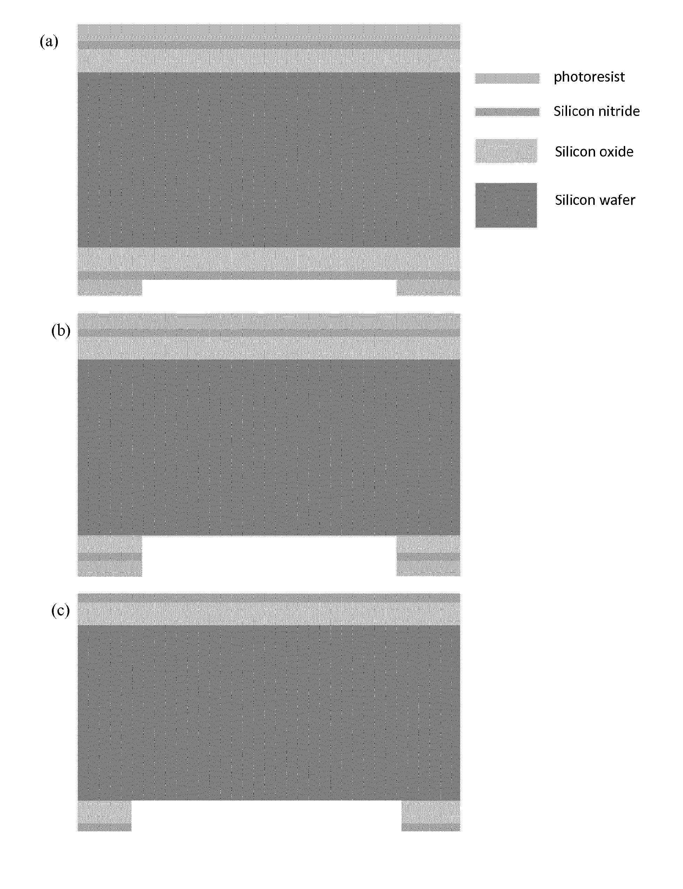

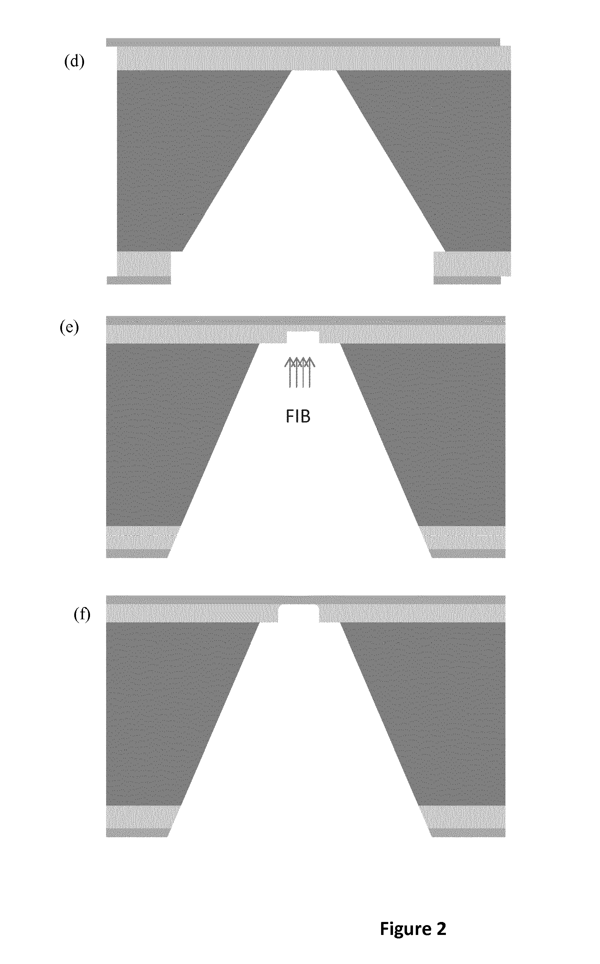

Figure 2 Schematic figure of the fabrication process of nanopore device (3 mm*3 mm chip). (a) Photoresist coating, exposure, development, lift off photoresist. (b) Etch through the silicon nitride and silicon oxide membrane of one side. (c) Lift off the photoresist. (d) Anisotropic etching through the silicon by KOH solution, leaving silicon oxide and silicon nitride membrane suspending on the other side. (e) Fabricate a small window of 1.5 µm in depth on the silicon oxide membrane with focus ion beam (FIB). (f) Etching away the rest of the silicon oxide membrane by RCA reaction, leaving the 2 µm*2 µm, and 70 nm thick silicon nitride membrane suspending.

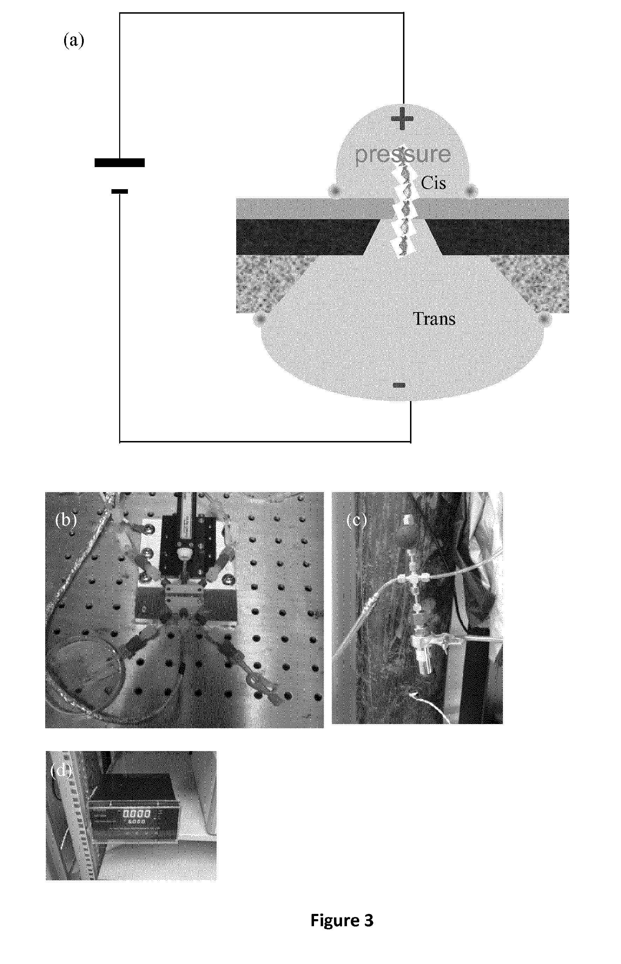

Figure 3 (a) Schematic diagram of ionic current measurement and external pressure introduction. (b) Photo of electrolytic cells and electrodes. (c) Photo of external pressure introduction. (d) Photo of Pressure display meter.

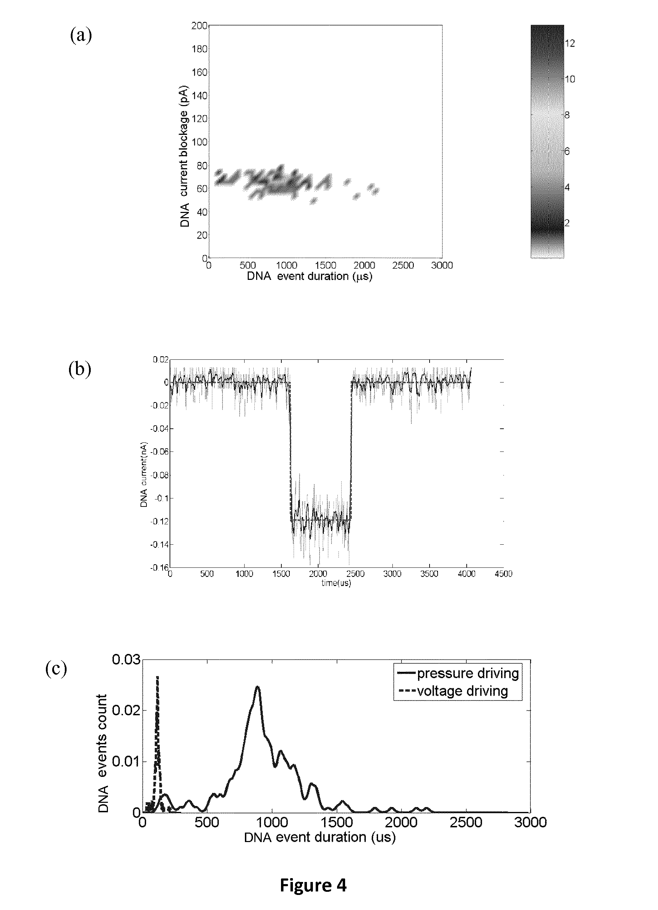

Figure 4 (a) Distribution diagram of DNA translocation current blockage-duration time, under the condition of 1.6 M KCl, 3 kb DNA and external pressure of 2.4 atm against 100 mV voltage bias. (b) Atypical DNA translocation event, with duration time of 800 µs. (c) The average translocation time of DNA through nanopore driven by external pressure is nearly 1 ms, which slows down the translocation speed by an order of magnitude compared with that driven by electric field only, about 100 µs.

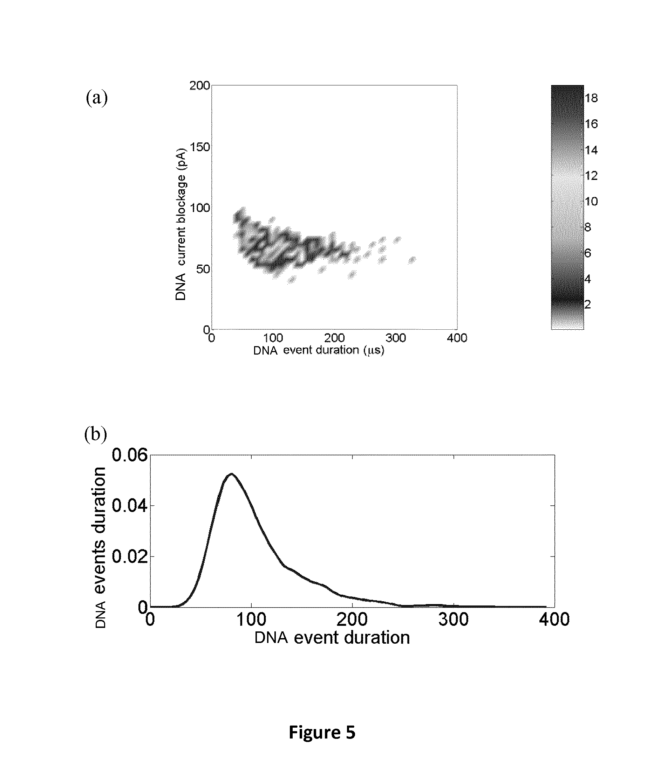

Figure 5 (a) Distribution diagram of DNA translocation current blockage-duration time, under the condition of 1.6M KCl, 600 bp DNA and external pressure of 2.4 atm against 100 mV voltage bias. (b) Distribution of event duration time.

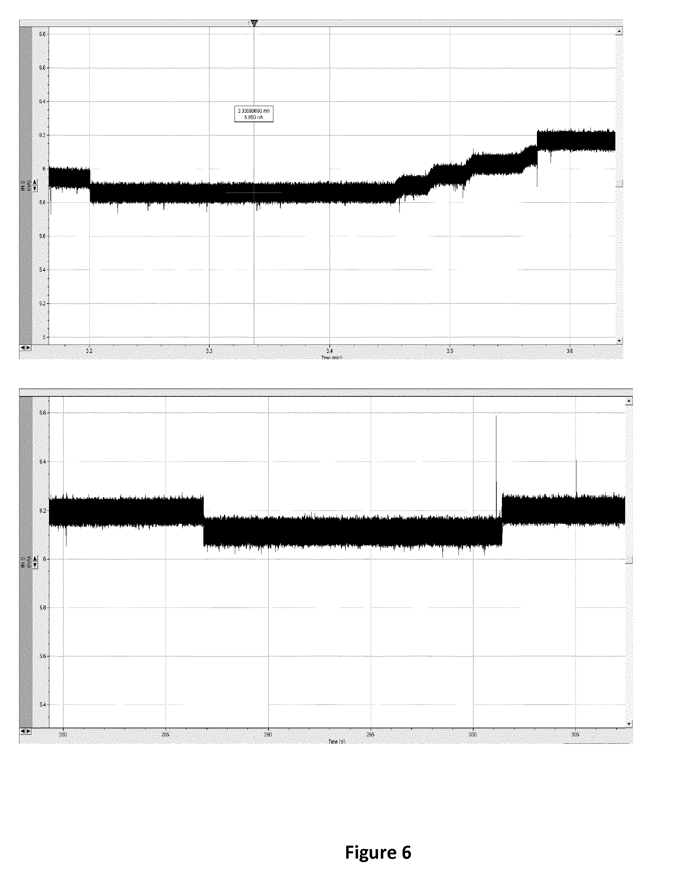

Figure 6 Display of ultralong DNA trapped event under the condition of 3 kb DNA, with external pressure of 2.0 atm against 100 mV voltage bias. The sudden blockage represent that DNA is trapped in the nanopore. (a) After 15 s of the ultralong time trapped event, when slowly adding up the voltage bias from 100 mV to 105 mV, the DNA molecule is pushed back to Cis chamber because of increase of electric force in nanopore, which leads to the current rise back to baseline. (b) After 15 s' retention in nanopore, the DNA molecule is pushed back to Cis chamber when the driving force by external pressure is decreased while slowly decreasing the pressure from 2.0 atm to 1.8 atm. We believe such trapped molecule is partly stuck or tethered on the membrane surface.

### DESCRIPTION OF THE INVENTION

The examples set forth below are a complete disclosure and description of how to make and use the preferred embodiments of the compositions, and are not intended to limit the scope of what the inventors regard as their invention.

Not otherwise specified, all the experiment methods are conventional, and all the experiment reagents and materials can be found in company.

### Examples

### 1) Chip device fabrication

The purpose of this procedure is to fabricate solid-state nanopore devices, which can be operated in transmission electron microscope (TEM). This process involved 2 separate steps. The first is to make freestanding SiN membrane devices, which can fit the TEM sample holder. The second is to drill nanopore on the freestanding SiN membrane with converged electron beam. This procedure includes multiple steps of standard micro-fabrication techniques in semiconductor industry, also involves some cutting-edge technology in nanofabrication field.

The details of the procedure: a low stress 150-200 nm-thick silicon nitride layer was grown by low pressure chemical vapor deposition on a silicon (100) substrate with 2µm-thick silicon dioxide. Using electron beam lithography and reactive ion etching (RIE), square windows about 584 *µ*m in size in the silicon nitride mask layer at one side were obtained. Figure 1(a) shows mask for photolithography, with arrays of 3×3 mm chips. Figure 1(b)-(c) shows the 584 µm transparent squares in the middle of the chips in figure 1(a). For the convenience and accuracy of wafer slicing process later and also for preventing the wafer cracking during wafer slicing process, small features with width 5 µm, length 20 µm were added, shown in figure 1(d). Mask parameters for photolithography: mask size: 5 inch; patterns: 100 mm in diameter; crystal direction indicator: 50 mm length, 0.8 mm width, 49 mm to the middle of the substrate, shown in figure 1(d).

Using photolithography (figure 2(a)) and reactive ion etching (RIE) (figure 2(b)), silicon dioxide layer and SiN layer were etched down to silicon wafer (figure 2(c)). Subsequently, pyramid-shaped holes were etched in the silicon wafer by KOH wet etching (40% KOH, 80°C, 6 hour). SiN layer in the holes should be flat and have no rough island structure observed under optic microscope, and around 20-80 µm, as shown in figure 2(d). Then slicing the wafer into 3×3 mm chips with protection of blue plastic. The detail procedure is: stick the wafer for slicing to another protection silicon substrate with wax at high temperature. Then stick blue plastic to the backside of the protection substrate. After slicing in 200-250 µm depth, remove blue plastic, protection substrate and wax. Each SiN/Si+ wafer (4 inches in diameter) was processed to produce more than eight hundred 3 × 3 mm² square chips. Phosphoric acid was used to etch SiN membrane to 70 nm thick with 1-5 nm/min etching rate at 160 °C, the thickness can be measured by ellipsometry. To reduce the capacitance noise in nanopore measurement and increase the survive chance in nanopore wetting, mini-membrane technique was employed here, shown in figure 2(e). Focused ion beam microscope (FIB, DB 235, FEI) was used to mill silicon dioxide to 0.5 µm thick in a 1*1 µm area. RCA reaction was used to remove the left 0.5 µm silicon dioxide layer, shown in figure 2(f). In this step, RCAreaction includes: NH₃·H₂O: H₂O₂ =1:1:5(v/v) to remove the organic contamination and hydrophilic for next step BOE, 10 min at 70 °C; BOE (HF: NH₄F: H₂O=1:2:3) 6min with etch rate 100 nm/min; HCl: H₂O₂: H₂O=1:1:5(v/v/v), 10 min at 70 °C to remove inorganic contamination.

### 2) Nanopore Drilling in TEM

This step is to fabricate nanopore in as prepared freestanding SiN membrane devices. Transmission electron microscope (FEI Tecnai F30) was used to drill nanopore at the following experiment condition: magnification 520k-890k, spot size:1. Nanopore with 10 nm in diameter and 60 nm-thick can be got within 5 min in this way. Sample holder was cleaned in plasma cleaner (O₂: Ar=1:3,v/v) for 1 min both before and after nanopore drilling. Nanopore device was stored in dry vacuum container afterwards.

### 3) Ionic current signal measurement under pressure force

This step is to detect ion current signal through nanopore under pressure force driving. Nanopore device was sealed by PDMS gasket and PEEK fluidic cells, and is the only channel connecting Cis and Trans chamber. Figure 3(a) shows how the structures of the whole experiment. Two electrodes (Ag/AgCl) were inserted in two chambers separately. Solution includes 1.6 M KCl, 1mM EDTA and 10 mM Tris (pH=8). 3 kb DNA molecules were injected to Cis chamber. Pressure was introduced between Cis and Trans chamber by connecting Cis chamber to a compressed nitrogen container, shown in figure 3(b)-(c). Figure 3(d) is a pressure monitor detecting pressure value in Cis chamber. By adjusting the pressure from compressed nitrogen container, pressure force between the two chambers was introduced to the system. Then ion current signal was measured at inverse voltage of 100 mV with the patch clamp system. Since DNA is negative charged in solution, inverse voltage can reduce DNA translocation speed through nanopore. By adjusting pressure and voltage, DNA molecule inside a nanopore can be manipulated controllably, for example, reduce translocation speed and stay inside nanopore for long time. Recent reports about nanopore-based DNA detection mainly focus on electric force driven technique, which will reduce the signal/noise ratio if decreasing voltage to reduce DNA translocation speed. Introducing pressure as driving force is a novel way in nanopore field, and is not reported in previous literatures. The advantage of using pressure as a driving force is to reduce DNA translocation speed, meanwhile maintain the excellent signal/noise ratio and high capture rate. Here two parameters were used to characterize single DNA molecule translocation event: current blockage and translocation time. Figure 4(a)-(b) shows 3kb DNA events detected at the experiment condition: pressure 2.4 atm; inverse voltage 100 mV, with average translocation time 800 µs and current 70 pA. Figure 4(c) shows the DNA events driven only by electric force with average translocation time 100 µs, almost one order of magnitude slower than normal electric force driving DNA events.

### 4) Reduce translocation speed of DNA molecules with different length

This step shows the effect of our invention on different DNA molecules. Here 600 bp DNA molecules were detected at the experiment condition: pressure 2.4 atm; inverse voltage 100 mV, with average translocation time 100 µs, shown in figure 5. Normally it's hard to detect 600 bp DNA using equipments available in the market now, due to the short length of 600 bp DNA and the relatively low time resolution of equipment. This clearly shows the advantage of our invention, successfully detecting shorter DNA molecules, which is hard for other techniques in the nanopore field.

### 5) Single molecule capture for ultra-long time

Besides the speed reducing effect by applying pressure driving force and counter voltage force, this step shows how to trap single molecule in nanopore for ultra-long time using our invention. DNA molecule can stay in nanopore for very long time if external force is balanced by adjusting inverse voltage and pressure, which can be used for single molecule trap inside nanopore. Here 3kb DNA translocation events with more than ten seconds translocation time can be found at pressure 2 atm and inverse voltage 100 mV, which has not been reported in other literatures. Figure 6 shows the capture and recapture of DNA molecules by adjusting pressure and voltage. When introducing 2 atm pressure, DNA molecules tethering on the SiN membrane were prone to stay in nanopore since external force on DNA molecule is almost balanced compared to the condition at 2.4 atm pressure, but is still slightly prone to pressure force direction. When external force changed, for example, pressure was decreased to 1.8 atm, DNA would be forced into Cis chamber. The same situation would happen if inverse voltage was increased to 105 mV. By adjusting pressure and electric force, DNA molecules can be manipulated controllably inside nanopore, which is hard for other techniques to achieve in the nanopore field.

### Industrial application

This invention, based on a conventional solid-state nanopore device, employs an easy technique, which introduces an external pressure force as the driving force for DNA translocation and an external voltage as the counter force, and effectively reduces DNA translocation speed more than one order of magnitude in a SiN solid-state nanopore with 10 nm diameter without losing signal-to-noise ratio. The time resolution of the device has been significantly improved, which will benefit DNA sequencing strategy based on solid-state nanopores in the future. By maintaining the detection signal with good signal-to-noise ratio and avoiding the uncontrollable interaction between DNA and nanopore surface within super tiny nanopore (less than 5nm in diameter), the repeatability and controllability of the experiment has been considerably improved. Moreover, by using appropriate solution concentration, voltage, and pressure, the detection of short DNA molecule less than 1kb can be achieved, which further expands the detection limit of solid-state nanopore on the length scale of molecule and reach the unexplored length scale for traditional solid-state nanopore device with the same pore size. The detection of DNA with short length is one of the main challenges for next generation sequencing work.

## Claims

1. A method to slow down nucleic acid molecule translocation through a solid-state nanopore comprising:
providing a flow cell setup containing an electrolytic solution, the flow cell setup including cis- and trans- chambers and a solid-state membrane separating the cis- and trans-chambers, the solid-state membrane including a nanopore;
introducing nucleic acid molecules to be detected into the cis- chamber of the flow cell setup;
applying pressure to the cis- chamber as a driving force field to translocate nucleic acid molecules through the nanopore; and
applying a voltage that generates an electric field from trans- to cis- chambers as a counter field to the applied pressure, during the translocation of nucleic acid molecules, with the applied pressure being large enough to generate a bigger pressure force than the electric force.

2. The method of claim 1 wherein the nucleic acid molecules comprise DNA, RNA, or Peptide nucleic acid molecules.

3. The method of claim 1 or 2, wherein the applied pressure is between 1.6 atm and 2.6 atm, and the applied voltage is between 40 mV and 160 mV.

4. The method of any one of claims 1 to 3, wherein the electrolyte has an electrolytic concentration of between 0.1 mol/L and 3.2 mol/L, a pH of between 8 and 10; the nanopore has a diameter of between 10 nm and 20 nm and the nanopore has a nanopore length of between 20 nm and 100 nm.

5. A method to trap a single nucleic acid molecule at a solid-state nanopore comprising:
providing a flow cell setup containing an electrolytic solution, the flow cell setup including cis- and trans- chambers and a solid-state membrane separating the cis- and trans-chambers, the solid-state membrane including a nanopore;
introducing nucleic acid molecules into the cis- chamber of the flow cell setup;
applying pressure to the cis- chamber; and
applying a voltage that generates an electric field from trans- to cis- chambers as a counter field to the applied pressure, with the applied pressure and applied voltage being selected to cause the net force on a nucleic acid molecule inside the nanopore to be zero or close to zero.

6. The method of claim 5 wherein the nucleic acid molecules comprise DNA, RNA, or Peptide nucleic acid molecules.

7. The method of claim 5 or 6, wherein the applied pressure is between 1.6 atm and 2.6 atm, and the applied voltage is between 40 mV and 160 mV.

8. The method of any one of claims 5 to 7, wherein the electrolyte has an electrolytic concentration of between 0.1 mol/L and 3.2 mol/L, a pH of between 8 and 10; the nanopore has a diameter of between 10 nm and 20 nm and the nanopore has a nanopore length of between 20 nm and 100 nm.

9. A method to control nucleic acid molecule motion direction comprising:
providing a flow cell setup containing an electrolytic solution, the flow cell setup including cis- and trans- chambers and a solid-state membrane separating the cis- and trans-chambers, the solid-state membrane including a nanopore;
introducing nucleic acid molecules into the cis- chamber of the flow cell setup;
applying pressure to the cis- chamber; and
applying a voltage that generates an electric field from trans- to cis- chambers as a counter field to the applied pressure, with the applied pressure and applied voltage being selected to produce a net force causing a nucleic acid molecule to translocate the nanopore, be trapped at the nanopore, be released at the nanopore, and reverse translocation through the nanopore.

10. The method of any one of claims 1 to 9, wherein the nucleic acid molecules have a length that is less than 1 kb.
